(19) Europäisches Patentamt
European Patent Office
Office européen des brevets

(11) **EP 2 279 171 B1**

(12) **EUROPEAN PATENT SPECIFICATION**

(45) Date of publication and mention
of the grant of the patent:
**23.09.2015 Bulletin 2015/39**

(21) Application number: **09753984.5**

(22) Date of filing: **02.06.2009**

(51) Int Cl.:
*C07C 403/24* (2006.01)   *A23K 1/16* (2006.01)
*A23K 1/18* (2006.01)   *A23L 1/275* (2006.01)

(86) International application number:
**PCT/EP2009/056749**

(87) International publication number:
**WO 2009/144329 (03.12.2009 Gazette 2009/49)**

(54) **CRYSTAL FORMS OF ASTAXANTHIN**

KRISTALLINE FORMEN VON ASTAXANTHIN

FORMES CRISTALLINES DE L'ASTAXANTHINE

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **30.05.2008 US 57337
30.05.2008 US 57332**

(43) Date of publication of application:
**02.02.2011 Bulletin 2011/05**

(73) Proprietor: **DSM IP Assets B.V.
6411 TE Heerlen (NL)**

(72) Inventors:
• **GUO, Jingfei
Halle-Wittenberg (DE)**

• **JONES, Matthew, J.**
**Halle-Wittenberg (DE)**
• **SCHAEFER, Christian**
**79618 Rheinfelden (DE)**
• **ULRICH, Joachim**
**Halle-Wittenberg (DE)**
• **VERLHAC-TRICHET, Viviane**
**F-68440 Dietwiller (FR)**

(74) Representative: **Schwander, Kuno et al
DSM Nutritional Products AG
Wurmisweg 576
4303 Kaiseraugst (CH)**

(56) References cited:
**WO-A-2007/020057   WO-A-2008/087090**

## Description

### Introduction

The invention relates to the field of crystal forms of astaxanthin. In particular, the invention relates to methods for producing said crystal forms.

In the feed industry, especially in the fish farming sector, astaxanthin is used for coloring, inter alia, salmon, trout and shrimps.

Astaxanthin is insoluble in water and only poor soluble in oil which means that solutions of astaxanthin for direct applications are unavailable. Furthermore astaxanthin is very sensitive to oxidation and heat treatment. Therefore, delivering astaxanthin with good oral bioavailability for improved plasma uptake and flesh deposition in salmonid is a particular concern for fish feed producers and fish farmers.

In order to make the colorant more bioavailable, several methods have been developed for preparing particulate astaxanthin compositions which are dispersible in water for processing into feed pellets. The dispersible compositions are prepared by dissolving crystalline astaxanthin in solvents (US 6,863,914 and US 6,406,735) or oils (US 5,364,563) under high pressure and temperature, immediately followed by dispersing the organic solution in aqueous hydrocolloid. Alternatively, the carotenoid is melted in an aqueous excipient-matrix and emulsified under pressure without using solvent or oil (US 6,093,348). All these methods require further processing to prepare powder or solid formulations from the aqueous dispersions.

WO 2007/020057 describes two crystal forms of astaxanthin and their potential utility for preparing astaxanthin formulations.

The inventors of the present patent application have found that it is of high importance in feed additive industries to choose the right polymorph (= crystal-form) of astaxanthin and that a specific crystalline polymorph of the molecule can be more useful than others.

"Right" means a polymorph of astaxanthin which is able to maintain best physicochemical properties of final formulations which is a key issue for product performance, such as dissolution rate, stability, solubility and bioavailability.

### Short Description of the Invention

It has been found that two specific crystal forms of astaxanthin show an improved bioavailability, a relatively high solubility in specific organic solvents and an increased long-term stability. For example the new forms are stable in solid form for at least 90 days at a temperature of 20°C - 40°C.

It was further found that the two crystal forms can be prepared from each other. Therefore the invention relates to methods for preparing said crystal forms.

In particular the present invention relates to the processes of making two crystalline forms of astaxanthin designated crystal form I and II, wherein

- crystal form I is characterized by the following parameters

  i) An XRPD (X-Ray Powder Diffraction) pattern comprising a peak between 20° and 21°, and
  ii) A DSC (Differential Scanning Calorimeter) scan showing a phase transition at 225°C - 235°C; and wherein

- crystal form II is characterized by the following parameters

  i) An XRPD pattern comprising a peak at approximately 11° and 18°, and
  ii) A DSC scan showing a phase transition at 200°C - 220°C.

Crystal form I is characterized in that it shows a phase transition at 230.8°C±1. In another example, crystal form I is stable in solid form for at least 5 months, preferably for at least 90 days at 20°C - 40°C.

Crystal form II is characterized in that it shows a phase transition at 210°C±1. In another example, crystal form II is stable in solid form for at least 90 days at 20°C.

In accordance with the present invention the following definitions apply:

"Astaxanthin compound" include astaxanthin molecules obtained during synthesis and crystallization of astaxanthin or during the extraction process of astaxanthin from natural sources.

"Mol%" indicates the purity of a crystal form with respect to total molar astaxanthin content of the astaxanthin compound.

"Lipophilic dispersing agent" is a solid substance with water solubility at room temperature lower than or equal to 5 mg/ml which has the property to embed a molecular or colloidal dispersion or aggregates of the astaxanthin in a solid composition.

"Hydrophilic dispersing agent" is a solid substance with water solubility at room temperature higher than 5 mg/ml which has the property to act as a wetting agent to enhance the suspension of astaxanthin in an aqueous phase.

"Solid composition" means that the astaxanthin is distributed in a solid matrix which is prepared by dissolving the carotenoid and the lipophilic or hydrophilic dispersing agent together in a mutual solvent or combination of solvents, followed by removal of the solvent or solvent mixture.

"Water miscible solvent" means that the solvent can be mixed in any ratio with water without phase separation, e.g. ethanol.

"Water immiscible solvent" means that the solvent can be mixed only partially with water without phase separation, e.g. dichloromethane.

"Crystallization liquid" is a liquid which is miscible with the solvent in which all-trans-astaxanthin is dissolved but has a lower solvency or practically no solvent properties (for astaxanthin) at the temperature that causes crystallization of the specific crystal form.

Crystals of astaxanthin according to the present invention derive from chemical synthesis well known to the person skilled in the art and are all-trans-astaxanthin.

Many techniques are used to identify the polymorphic forms of a material and its relative temperature stability. These techniques include X-ray powder diffraction (XRPD), thermogravimetric analysis, differential scanning calorimetry (DSC), RAMAN spectroscopy, optical and electrical microscopy and UV-VIS techniques.

In most cases X-ray diffractometry is capable to reflect the differences in crystal structure. Ideally, X-ray diffraction on a single crystal (typical dimensions 100x100x100 $\mu$m$^3$) yields a three-dimensional diffraction pattern of, normally, well-resolved peaks, which after phasing can be back-transformed into electron density. As a matter of fact, it is often difficult, if not impossible, to obtain single crystals of required quality and size from a given material. In powder diffraction experiment the sample consists of a huge number of crystallites with typical dimensions of 5x5x5 $\mu$m$^3$. The powder is normally obtained by grinding or milling. In case of crystal form I and II, XRPD patterns are obtained by powder diffraction experiments.

Thermal analysis methods are defined as those techniques in which a property of the analyte is determined as a function of an externally applied temperature. In many respects, DSC is an easy method to use routinely on a quantitative basis, and for this reason it has become a widely accepted method for identification and characterization. The field of thermal analysis and the level of understanding of polymorphism have both grown rapidly in recent years.

In accordance with the present invention, a DSC (Netzsch Phoenix 204) is used to investigate the thermodynamic relationship between different polymorphs. The DSC cell is calibrated with indium (T$_m$ = 156.6 °C, $\Delta$H$_{fus}$=28.54J.g.$^{-1}$). Astaxanthin (normally 3 - 6 mg) was heated up at certain rate, from 20°C - 250 °C in Al-pans while being purged with nitrogen.

Raman spectroscopy is a kind of vibrational spectroscopy. It is commonly used in chemistry, since vibrational information is specific for the chemical bonds in molecules. It therefore provides a fingerprint by which the molecule can be identified. In accordance with the present invention, Brucker FT-Raman spectroscopy RFS 100S is used. An initial laser is set to 1284 mm$^{-1}$. Stainless sample holder is used with amount of several micrograms. Finally data are analyzed by the software of Origin and OPUS.

As mentioned above, crystal form I is characterized by

i) An XRPD pattern comprising a peak between 20° and 21°, and
ii) A DSC scan showing a phase transition at 225°C - 235°C;

Furthermore crystal form I shows a solubility profile in dichloromethane of 0.1 - 0.2 g/ml at 20°C - 25°C.

As mentioned above, crystal form II is characterized by

i) An XRPD pattern comprising a peak at approximately 11° and 18°, and
ii) A DSC scan showing a phase transition at 200°C - 220°C.

Furthermore crystal form II shows a solubility profile in dichloromethane of 0.3 - 0.4 g/ml at 20°C - 25°C.

A preferred embodiment of an astaxanthin compound is consisting essentially of 50% to 100% by weight of the crystalline form I or II.

An other preferred embodiment of an astaxanthin compound relates to a mixture consisting essentially of 95% to 5% by weight of the crystalline form I and 5% to 95% by weight of the crystalline form II.

As mentioned above, the invention relates to a process for the transformation of crystal form I of astaxanthin into crystal form II of astaxanthin and vice versa.

With regard to the transformation of crystal form I of astaxanthin into crystal form II, the process is characterized in that

a) crystal form I is heated to below its melting point and then quenched to room temperature or
b) crystal form I is dissolved in a solvent characterized by a vapor pressure of > 20 kPa and then the solvent is evaporated, or
c) the transformation is carried out by slurry conversion in an organic solvent as for example chloroform.

With regard to the transformation of crystal form II of astaxanthin into crystal form I, the process is characterized in that the transformation is carried out by slurry conversion in an organic solvent as for example dichloromethane or ethyl acetate.

Crystal form I or II or mixtures thereof for use in the life science industry, especially for use in the fish feed industry have to be formulated into a suitable administration form.

For this purpose the astaxanthin crystal form I or II or mixtures thereof may be

- dissolved in an organic solvent or oil or mixtures thereof followed by further processing into said administration form;
- dissolved in an organic solvent or oil or mixtures thereof followed by further processing into said administration form which comprises a lipophilic dispersant;
- dissolved directly in an edible oil and/ or fish oil at temperatures between 100°C and 230°C for direct incorporation in fish feed pellets and other application forms.

In particular, a process for preparing a stable water-dispersible administration form of astaxanthin for use in the nutrition industry is disclosed. The process is characterized by dissolving astaxanthin crystals of form I or II in an organic solvent, mixing this solution with an aqueous solution and converting the dispersion which has formed into a water-dispersible dry powder by removing the solvent and the water and by drying in the presence of a coating material without changing the crystal form.

## Short Description of the Figures

Figure 1 shows an X-Ray diffractogram of crystal form I and II of astaxanthin.

Figure 2 shows a Raman spectra of crystal form I and II of astaxanthin.

Figure 3 shows a Raman spectra of two formulations of crystal form I and II in the range from 500 to 1000 cm$^{-1}$ compared with a control.

## Detailed Description of the Figures and of Examples

The preferred method for obtaining astaxanthin is to utilize chemical synthesis such as described in US 5,654,488. Synthetic astaxanthin is a 1:2:1 mixture of the diastereoisomers (3S,3'S), (3R, 3'S) and (3R, 3'R). A further method for obtaining astaxanthin is via fermentation, or from microalgae as disclosed for example in WO-89/1997 and EP329754 or from yeast Phaffia rhodozyma.

The known art for preparing astaxanthin formulations for fish feed does not disclose the possibility of preparing specific crystal forms clearly characterized by XRPD and DSC in addition to at least one other physical parameter such as Raman spectrum, solubility in organic solvents. The crystal forms have different solubility in organic solvents and oils which enable a wider choice for handling and formulating astaxanthin compositions. Furthermore, the crystal forms as defined above affect in vivo dissolution rate and allow higher (supersaturated) concentrations in administration forms which provide higher uptake and bioavailability, after oral administration.

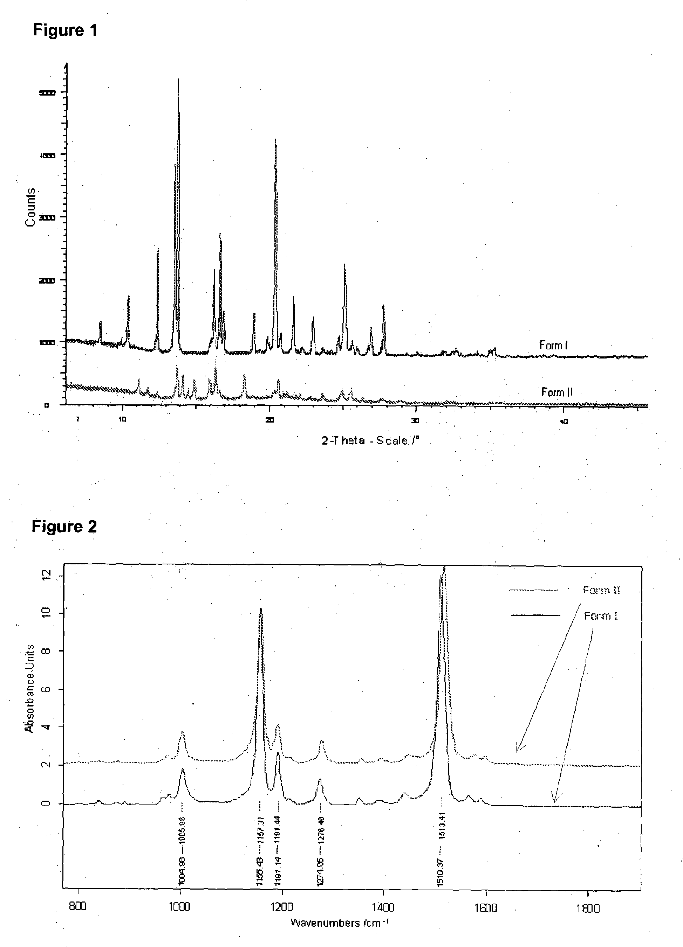

The X-ray powder diffraction patterns as shown in figure 1 were obtained by collecting intensity data measured by a Bruker D4 diffractometer. The system is equipped with a Cu anode and a monochromator providing Cu-Kα1 radiation (A = 1.54056 A). Measurements were carried out using a step width of 0.005° and an acquisition time of 4 s per step.

Figure 1 shows the pattern of the crystal form II compared with the form I. The pattern of the crystals shows different characteristic peaks in the scanning range. Especially, the high intensity peak observed at 20.35° in form I is absent in the pattern of form I Also, two peaks observed at 29 = 11.07° and 18.32° of the form II do not match the pattern of form I.

DSC was used to investigate the thermodynamic properties of the two different polymorphs. The DSC cell was calibrated with indium (Tm= 156.6°C, $\Delta$Hfus= 28.54 J·g$^{-1}$).

To investigate the different polymorphs, 3-5 mg samples of form I or II were heated from 20 °C to 250 °C at a heating rate of 5 K·min$^{-1}$, respectively, while being purged with nitrogen (10 ml·min$^{-1}$).

The DSC analysis shows one endothermic event between 220 °C and 235 °C for form I and another in the range between 200 °C and 220 °C for form II, recognized as melting of the samples. In addition, another small endothermic peak at 216.6 °C is also observed on the heating trace of form I.

Furthermore, resonance Raman spectroscopy (FT-Raman-Spectrometer RFS 100/S, Bruker) was used to investigate the polymorphism of astaxanthin. The measurement was performed on a FT Raman spectrometer with Raman microscopy and temperature control unit. The laser operated at 1024 nm with back scattering optics.

The resonance Raman spectra of astaxanthin form I and II are shown in figure 2 and table 1.

Table 1: Results of resonance Raman spectroscopy

| Name | CH$_3$ cm$^{-1}$ | C-H cm$^{-1}$ | C=C cm$^{-1}$ |
|---|---|---|---|
| Form I | 1004.98 | 1155.43 | 1510.37 |
| Form II | 1005.98 | 1157.31 | 1513.41 |

The most intense band at 1510 cm$^{-1}$ is assigned to the C=C stretch vibrations of the polyene chain. The second most intense band at 1155 cm$^{-1}$, which represents the superposition of two modes that can be ascribed to C-H in-plane bending vibrations mixed with C-C stretching and C=C-C bending vibrations, respectively. The third intense line at 1004 cm$^{-1}$ can be assigned to CH$_3$ in-plane rocking vibrations.

A number of shifts and differences of the spectral bands can be seen that distinguish the polymorphs. In the region from 950-1000 cm$^{-1}$, for instance, form II has only one sharp band whereas form I shows two bands.

Crystal form I and II may be prepared from a solution with highly pure astaxanthin, (i.e. greater than 95%) followed by treatment of the solution with e.g. heat and/ or light to cause the formation of a sufficient amount of astaxanthin compound to obtain essentially crystal form I and/or form II after final crystallization. It should be understood that the aforementioned crystallization methods may be carried out preferably after synthesis for preparation of either form I or form II crystals. Alternatively, they may also be carried out in the purification or extraction steps for astaxanthin crystals (as part of chemical synthesis or isolation of the astaxanthin compound from natural products) wherein at least one crystallization step for preparing astaxanthin crystal, or a (re)crystallization procedure utilizing solvents is employed.

There are several crystallization methods based on similar principles that may be considered to prepare crystal form I, form II or mixtures comprising form I and form II, starting from solutions (In general, suitable solvents to prepare the solutions are solvents which dissolve at least 1 mg/ml, preferably up to 10 -50 mg/ml of astaxanthin at the temperature when crystallization is initiated.) differing in compound profile and concentration. Suitable crystallization methods that may be considered by a skilled person include but are not limited to the following methods.

Starting from an apolar aprotic organic solution of astaxanthin under controlled temperature conditions, crystallization is induced by removal of the solvent from the solution, optionally by simultaneous exchange with a miscible polar crystallization liquid. A preferred apolar aprotic solvent is dichloromethane. Alternative chlorinated apolar aprotic solvents are e.g. chloroform, trichloroethane. Suitable non-chlorinated alternatives are dimethoxymethane, diethoxyethane and dioxacyclopentane. A preferred polar crystallization liquid is methanol or other alkanols such as ethanol, n-propanol, isopropanol, n-butanol and tert-butanol.

Crystals comprising form I or II may also be obtained by removal of the solvent from an astaxanthin solution in a polar aprotic or polar protic solvent by evaporation. Solvents which have a high solubility for astaxanthin and a low boiling point are preferred.

Another method to induce crystallization is cooling of an (over) saturated solution in an apolar aprotic solvent. Preferred apolar aprotic solvents are dichloromethane, toluene or alternative chlorinated apolar aprotic solvents. Polar solvents such as tetrahydrofuran (THF), N-methylpyrrolidone (NMP), N-ethylpyrrolidone (NEP) and pyridine which have a high solubility for astaxanthin may be considered as well. The crystallization rate and yield of the desired crystal form may be increased by adding seeds of the pure form to the crystallizing solution. Crystals comprising form I or II can also be obtained by dilution of a solution of astaxanthin containing the desired concentrations of all-trans-astaxanthin in a apolar aprotic, polar aprotic or polar protic solvent by adding a miscible polar crystallization liquid. Examples of apolar aprotic solvents are dichloromethane, toluene or alternative chlorinated solvents. Crystallization liquid is in that case an alkanol like methanol. Examples of suitable polar solvents are THF, NMP and pyridine.

The resulting crystals are harvested by e.g. filtration, spontaneous sedimentation or centrifugation methods known in the art, optionally washed with a suitable solvent, preferably with a cold alkanol (preferably methanol) and dried, preferably under vacuum. The resulting crystals may be milled to obtain the desired particle size for further processing.

Mixtures of crystal form I and II may contain from 5 % to 95 % of form I and 95 % to 5 % of form II, or from 20 % to 80 % of form I and 80 % to 20 % of form II.

Crystal form I, form II and mixtures of the two forms are suitable for incorporation as such in solid, semi solid and liquid (oily-) formulations suitable for administration to an organism. Preferred examples of solid forms are, granulates, pellets, powders, etc. Preferred examples of semi-solid forms are suspensions. They particularly include particulate microns suspensions of crystal form I or form I or mixtures of form I and II in an oily vehicle.

Reference examples of administration forms which may be prepared using crystal form I and/or II are oil dispersible compositions as for example described in WO03/102116. Crystal form I or form II or mixtures thereof may also be used for preparing water-dispersible compositions as described in US 2,861,891, US 5,364,563 and US 6,296,877.

The usual method to prepare solid compositions and formulations comprising astaxanthin is to dissolve the crystal form I, form II or mixtures thereof in an organic, water miscible or water immiscible solvent or mixtures thereof in the presence of suitable excipients, followed by removal of the solvent by either dilution in water or evaporation techniques as it is described in WO03/102116. Crystal form I or II may be directly employed as such and dissolved in oily solutions of astaxanthin by applying energy.

The solvents used to prepare solutions and for processing of the astaxanthin crystal form I or II or mixtures thereof into dry astaxanthin compositions may be water miscible or water immiscible. Examples of water miscible and immiscible solvents include the examples of solvents used for crystallization of the crystal forms that are listed above. By the application of heat/pressure, a crystallization liquid employed during crystallization under normal pressures and ambient temperatures may be used as solvent for astaxanthin (e.g isopropanol/ water). Preferred examples of excipients are dispersants, polymers and synthetic natural gums and cellulose derivatives which may be either hydrophilic or lipophilic.

The solid astaxanthin composition comprises between 2.5 wt % to 25 wt %, preferably 5 wt % to 15 wt %, of total astaxanthin. The amount of dispersant used in the composition is preferably between 50 wt% to 97.5 wt %. Varying amounts of excipients may be used as bulking agents to make up the final form.

Suitable lipophilic dispersing agents may be selected from particular members of the group consisting of ethylcelluloses, synthetic and natural resins, rosins and gums.

Suitable hydrophilic dispersants include but are not limited to protective colloids of low- and high-molecular-weight components of, for example, gelatin, fish gelatin, starch, dextrin, plant proteins, pectin, gum arabic, casein, caseinate or mixtures thereof; the protein-containing protective colloids, in particular non-gelling low-molecular-weight protein hydrolysates and higher-molecular-weight gelling gelatins being preferred. Further hydrophilic dispersants may be selected from members of the group consisting of PEG (polyethylengylcol), polyvinylpyrrolidone, polyvinylalcohol, polyvinylpyrrolidone-polyvinylacetate copolymer, hydroxypropylmethylcellulose (HMPC), hydroxypropylcellulose (HPC), hydroxypropylmethylcellulose phthalate (HPMCP), polyacrylates and polymethacrylates.

A preferred reference example of a solid formulation of astaxanthin comprises

a) a matrix substance forming an outer (continuous) phase; and
b) inner (discontinuous) phase within said matrix substance which comprises

1) the astaxanthin form I or II which is embedded in
2) a physiologically acceptable encapsulating substance which is solid at room temperature and, together with astaxanthin, is homogeneously soluble in an organic solvent.

The term "encapsulating substance" denotes any edible substance, that is solid at application temperature, able to encapsulate the active ingredient and soluble together with the active ingredient in one common solvent. Preferably used are substances, which are commonly used as coating materials. More preferably used are synthetic or natural waxes or wax-like substances, or natural or synthetic edible polymers.

The wax or wax-like substance is preferably selected from among e.g. carnauba wax, candelilla wax, beeswax, rice bran wax, sugar cane wax, cork wax, guaruma wax, ouricury wax, montan wax, spermaceti, lanolin, paraffin wax, fats, hydrogenated fats, fatty acid monoglycerides, polypropylene glycol, polyethylene glycol, and fatty acid esters.

Natural edible polymers are preferably selected from modified (e.g. alkylated) carbohydrates (e.g. starch, pectin, alginate, carrageenan, furcellaran, chitosan, maltodextrin, dextrin derivatives), celluloses and cellulose derivatives (e.g. cellulose acetate, methyl cellulose, hydroxypropyl methyl cellulose) and gums or modified (e.g. alkylated) gums (e.g. gum arabic, gum xanthan, gum guar, gum ghatti, gum karaya, gum tragacanth, locust bean gum, gellan gum). Modification of these polymers may be necessary to improve solubility in organic solvents.

The synthetic polymer is preferably selected from among the synthetic waxes such as polyethylene and polypropylene waxes, coumarene-indene resins, polylactic acid (PLA) and poly(lactic/glycolic) acid (PLGA), acrylic polymers (methacrylic acid copolymers and ammonio methacrylate copolymers), polyorthoesters, polyphosphazenes, polyanhy-

drides, polyglycolide (PGA), poly($\varepsilon$-caprolactone), polydioxanone, trimethylene carbonate, poly($\beta$-hydroxybutyrate), poly($\gamma$-ethyl glutamate), poly(DTH iminocarbonate), poly(bisphenol A iminocarbonate) and polycyanoacrylate, especially the acrylic polymers.

Matrix components are preferably selected from among carbohydrates (e.g. cellulose, starch, modified starch, dextrin, pectin, alginate, carrageenan, furcellaran, chitosan), gums (e.g. gum arabic, gum xanthan, gum guar, gum ghatti, gum karaya, gum tragacanth, locust bean gum, gellan gum), proteins (e.g. fish, poultry and mammalian gelatine, soy protein, pea protein, zein (from corn) wheat gluten, lupin protein, peanut protein, milk proteins or hydrolysed or modified milk proteins, especially casein or whey proteins, lignins and lignin derivatives (e.g. lignosulfonates, kraft lignins), celluloses and cellulose derivatives (e.g. carboxymethyl cellulose, carboxyethyl cellulose, hydroxypropyl cellulose).

Preferred matrix substances are gelatin, lignosulfonates, milk proteins or hydrolysed milk proteins, plant proteins or hydrolysed plant proteins, or modified starch, especially gelatine, casein and casein hydrolysates, soy protein, hydrolysates thereof, lignosulfonate, physically modified soy protein, starches and modified starches, especially octyl succinyl starch, pectins and carboxymethyl cellulose.

Particularly preferred are matrix substances which provide cold-water soluble compositions, such as lignosulfonate, fish gelatin, milk protein and hydrolysed plant proteins.

As solvent any organic solvent or solvent combination may be used that is able to dissolve astaxanthin compounds. Volatile solvents and solvent combinations that are easy to evaporate from the emulsion are preferred. Examples for solvents are isopropanole, hexane, cyclohexane, acetone, methyl ethyl ketone, methylene chloride, chloroform, toluene, tetrahydrofurane, acetic acid ethyl ester.

As will be apparent from the foregoing, the preferred solid composition comprises a matrix substance as a continuous phase wherein particles (droplets) of an encapsulating substance are distributed. Within said particles (droplets) of the encapsulating substance, astaxanthin is distributed. Such compositions are distinguished from compositions wherein particles of astaxanthin are distributed within a matrix substance (see, e.g. EP 564 989) or compositions wherein astaxanthin is coated with a coating material.

**Examples**

The following representative examples illustrate methods to prepare astaxanthin crystal forms I and II and methods to incorporate astaxanthin crystal form I and/ or crystal form II in administration forms. The examples also illustrate that the two forms have a relatively high solubility in specific organic solvents and an increased long-term stability which is of advantage in the feed industry. Finally the examples also illustrate the use of crystal form I or crystal form II or defined mixtures thereof in administration forms for improving the stability of the forms and for improving the bioavailability of astaxanthin, i.e. for providing higher oral uptake of the compound.

Example 1: Preparation of crystal form I

For preparing the desired crystal form according to method described above, astaxanthin from Sigma (natural astaxanthin) and Dr. Ehrenstorfer (synthetic astaxanthin, analytical grade) may be used. The purity of the compound can be determined by HPLC measurements and characterised by X-ray diffraction and Raman spectroscopy. Ultra pure astaxanthin may also be obtained from less pure astaxanthin by means of preparative HPLC.

Example 2: Preparation of crystal form II from crystal form I

Form II can be prepared by the heat treatment or evaporation from certain solvents, such as acetone. Due to the low solubility (approximately 8 mg·100ml$^{-1}$ in acetone at 20.5 °C), only a very small amount of crystals can be obtained by evaporation. Heat treatment was therefore used as the main method for crystal preparation.

Form II was produced by heating form I to just below its melting temperature and then quenching. Heating of form I was carried out in a DSC (Netzsch Phoenix 204) cell. Approximately 6-7 mg astaxanthin form I were heated in the Al-pan at a heating rate of 5 K·min-1, from 20 °C to 200 °C, and then slowly heated up to 224 °C at a heating rate of 2 K·min-1. Immediately after this temperature was reached, the sample was cooled down from 224 °C to 20 °C at 40 K·min-1. The DSC system was purged with nitrogen throughout. XRPD and DSC curves confirm the second crystal form of astaxanthin.

Different analytical techniques were used to investigate the polymorphs. XRPD proved the existence of form II as defined above.

Thermal analysis showed form I melting at 230.4 °C while form II melts at 216.7 °C. Finally, Raman spectra provided sufficient information to identify the polymorphs. Differences in peak position and shifts of Raman bands between form I and II make it possible to distinguish both forms at ambient condition.

Example 3: Solubility measurements

For the pharmaceutical and nutritional compounds, solubility of different polymorphs in various solvents has attained an increased interest in recent years.

The solubilities of astaxanthin form I and II in different solvents were compared. Due to the very low solubility in some solvents (isopropanol and corn oil), experiments in these two solvents were measured by using an UV-VIS meter. The saturated solutions were prepared at different temperature in a double jacked vessel and 1 ml of the solution was taken out and measured by the UV-VIS meter. To calculate the real concentration in these solvents, the calibration was made firstly by building up the correlation between absorbance unit and solution concentration.

The experiments showed that the solubility differs markedly depending on the solvent. For example, the solubility of form II in methylene chloride (DCM) exceeds 280 mg·100 ml$^{-1}$ whereas the solubility in isopropanol is only 3.4 mg·100 ml$^{-1}$ at 30°C. A higher solubility of form II can be observed in these solvents.

The ideal solubility can be estimated by using the equation with measured melting points and heat of fusion form DCS data. Results are summarized in table 2.

Table 2. Ideal solubility calculated of forms I and II in DCM

| Exp. | T °C | 1/T 1/K | Conc. form I g/mL | Conc. form II g/mL | Ln X form I | Ln X form II | Ratio(Conc.) |
|---|---|---|---|---|---|---|---|
| | 12,00 | 0,00 | 0,01 | 0,02 | -6,71 | -6,19 | 1,68 |
| | 21,80 | 0,00 | 0,01 | 0,02 | -6,56 | -5,96 | 1,83 |
| | 30,70 | 0,00 | 0,02 | 0,04 | -5,95 | -5,52 | 1,54 |
| Cal. | T °C | 1/TK 1/K | Conc. form I g/mL | Conc. form II g/mL | Ln X form I | Ln X form II | Ratio (Conc.) |
| | 12,00 | 0,00 | 0,00 | 0,02 | -14,17 | 6,00 | 3530,79 |
| | 21,80 | 0,00 | 8,00 | 0,04 | -13,08 | 5,52 | 1927,23 |
| | 30,70 | 0,00 | 0,00 | 0,06 | -12,16 | -5,12 | 1151,20 |
| | 39,00 | 0,00 | 0,00 | 0,08 | -11,34 | -4,76 | 731,67 |

The solubility ratio typically decreases when the temperature increases (unless there is an enantiotropic transition between the temperatures of solubility determination in the higher temperature of interest). The ratio of determined solubility between astaxanthin form I and II is in the range form 1.5 to 1.8.

Example 4: Solvent-mediated transformation - slurry conversion experiment

Solvent-mediated polymorphic transformation is an efficient technique to study the phase stability of different crystal forms in various solvents. In this technique, the less stable form is suspended in a saturated solution of the solvent which is of interest. The more stable form will then crystallize at the expense of the less stable form, because the apparent solubility of this metastable form is higher than the solubility of the most stable form. As transformation rates in different solvents vary from minutes to years, an appropriate solvent should be chosen to either facilitate or retard the transformation.

Currently, the choice of a solvent is still done by trial and error, which is time consuming. In accordance with the present invention, the stability test was carried out by the slurry conversion experiments in six different solvents. Form II (and in one case form I) is suspended as the initial form and the resulting crystals were analyzed by XRPD.

Table 3. Results of slurry conversion experiments in 6 solvents

| Solvent | Time | Temperatures /°C | Solubility /mg· 100ml$^{-1}$,20°C | Initial form | Final form |
|---|---|---|---|---|---|
| EtOH | 4 days | 40 | 11,8 | II | II |
| Acetone | 4 days | 40 | 9.0 | II | II |
| DCM | 1 day | 20 | 1082.0 | II | I |
| I PA | 4 days | 40 | 6.0 | II | II |
| Chloroform | 1 day | 20 | 200.0 | I | II |
| EtOAc | 4 days | 40 | 10.0 | II | I |

The two forms can be prepared via solvent-mediated transformation. Form the results listed in table 3, it can be seen that the transformation of form II into form I is only detected in EtOAc (ethyl acetate) and DCM (methylene chloride) and that the transformation of form I into form II is only detected in chloroform.

Reference Example 5: Oily form of crystal form I or II

10 g of astaxanthin crystal form I or form I are mixed with 90 g soy bean oil. The crystals are milled and the resulting micronised suspension is suitable for preparation of powder formulations or can be directly dissolved in oil using a flash heating procedure followed by cooling with an excess of an oil phase or water phase comprising an emulsifier.

Reference Example 6: Colloidally dispersed formulation of forms I and II

In a heatable receiving flask, 4 g of astaxanthin crystal form I or form II and 1.5 g of peanut oil are suspended in a solution of 1.2 g of ethoxyquin in 29 g of isopropanol/ water (88/12, w/w) at 30°C. This suspension is mixed at a mixing temperature of 170[μ] with 59 g of isopropanol/ water (88/12, w/w) with a residence time of 0.2 seconds. The resulting molecularly dispersed astaxanthin solution immediately afterward enters a further mixing chamber. 11.3 g of an aqueous gelatin solution, adjusted to pH 9 which, in addition to 8.4 g of gelatin A (100 Bloom, M. W. =94,000), containing 4.2 g of Gelita Sol P (M. W. =21,000) and 9.2 g of sucrose, is added to precipitate the astaxanthin, at 45°C, in colloidally dispersed form.

Reference Example 7: A powder composition

The powder composition is prepared by dissolving 1 g the astaxanthin crystal form I, with 8 g ethylcellulose N4 (The Dow Chemical Company) and 1 g alpha-tocopherol in 90 g dichloromethane (Fluka), followed by removal of the solvent to produce a granulate, using spray granulation.
Another powder composition is prepared by dissolving 0.80 g the astaxanthin crystal form II with 8.4 g ethylcellulose N4 (The Dow Chemical Company) and 0.80 g alpha-tocopherol in 90 g dichloromethane (Fluka), followed by removal of the solvent to produce a granulate, using spray granulation.

Reference Example 8: Astaxanthin beadlet

15 g of an astaxanthin compound of form II and 15 g beeswax are dissolved together with 3 g Ethoxyquin in 600 ml chloroform. 75 g Na-Lignosulfonate is dissolved in 375 ml demineralised water. The pH of this solution is adjusted to $7.5 \pm 0.5$ using a 20% w/w sodium hydroxide solution. The oil phase is added slowly to the aqueous phase using both a high rate of mixing and a high shear force mixer. After the addition is completed, the emulsion temperature is maintained at 50° C. while high speed shear mixing is continued for 15 minutes. The temperature is gradually raised and mixing is continued until all the chloroform has been evaporated. This evaporation is usually completed when the temperature of the emulsion reaches about 75° C. During the evaporation procedure, distilled water is added to the emulsion to maintain a suitable viscosity.
After all the chloroform has been removed, distilled water is added and thoroughly admixed with the emulsion to achieve an emulsion solids content and viscosity suitable for spraying. The emulsion is then sprayed into a bed of 1 kg of fluidized starch using a lab spraying-pan. Residual starch is removed by sieving.

Reference Example 9: Stability of forms I and II

Two solid formulations of the crystal forms I and II were prepared according to methods mentioned above. The formulations have been analyzed by Raman spectroscopy and - with regard to stability - by UV retention, a commonly used method for stability measurements.
The samples used in this experiment are:

Sample A:  crystal form I prepared with methylene chloride
Sample B:  crystal form II prepared with chloroform
For both formulations a matrix-composition consisting of a $H_2O$-phase (Ca-Lignosulfonate & Yellow dextrin) and a Oil-phase ($CH_2Cl_2$) have been used.
Sample C:  As a control commercial product Carophyll Pink 10%-CWS from DSM Nutritional Products Ltd was used.
In this case the following matrix-composition was used: $H_2O$-phase: Ca-Lignosulfonate & Yellow dextrin; Oil-phase: $CH_2Cl_2$, & d,l-$\alpha$-tocopherol, bees wax.)

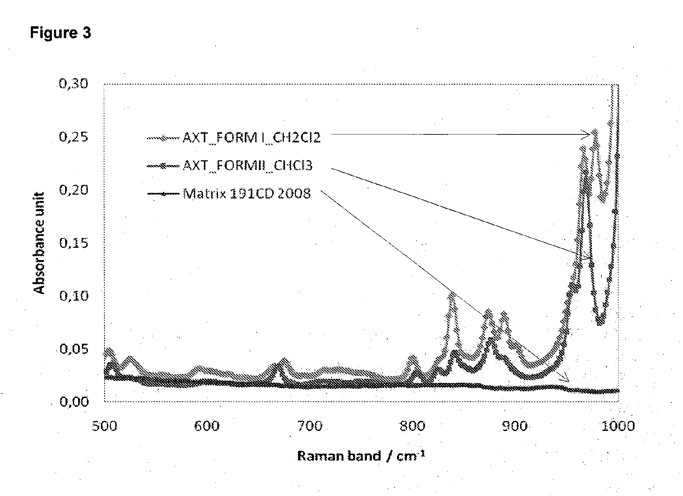

Figure 3 shows the Raman spectra of the two formulations of crystal form I and II in the range from 500 to 1000 cm$^{-1}$ compared with the control. The profiles clearly indicate that the two crystal forms as used for the preparation of the solid formulation samples are still present in the final form.

The results of the stability test are shown in tables 4 and 5. The results indicate that the formulated crystal forms are stable in solid form for at least 90 days at 20°C.

Table 4 Stability measurements (40°C, UV retention - (%)) : Sample A

|  | Start | 1 month | 2 months | 3 months | 6 months |
|---|---|---|---|---|---|
| all-E astaxanthin | 97 | 95 | 95 | 95 | 94 |

Table 5 Stability measurements (40°C, UV retention - (%)) - Sample B

|  | Start | 1 month | 2 months | 3 months | 6 months |
|---|---|---|---|---|---|
| all-E astaxanthin | 84 | 79 | 78 | 78 | 80 |

Reference Example 10: Experimental design for determination of bioavailability

Determination of the bioavailability of astaxanthin from different formulations can be done by calculating the apparent digestibility coefficients (ADC).

For example, ADC of astaxanthin can be calculated as a fractional net absorption of nutrients from diets based on yttrium oxide ($Y_2O_3$) as a non absorbable indicator.

ADC can be calculated using the following formula:

$$ADC \text{ of nutrient} = 100 - [100 \times (\%Y_2O_3 \text{ in feed}/\%Y_2O_3 \text{ in faeces}) \times (\% \text{ nutrient in faeces}/ \% \text{ nutrient in feed})]$$

The digestibility data can then be transformed in arc sinus of square root of percent values before being subjected to one-way ANOVA analysis. Finally, StatBoxPro software (Grimmersoft, version 5.0) can be used to perform statistical calculations.

**Claims**

1. A process for the transformation of crystal form I of astaxanthin into crystal form I of astaxanthin, wherein

   • crystal form I is **characterized by** the following parameters

   i) An XRPD (X-Ray Powder Diffraction) pattern comprising a peak between 20° and 21 °, and
   ii) A DSC (Differential Scanning Calorimeter) scan showing a phase transition at 225°C - 235°C; and wherein

   • crystal form II is **characterized by** the following parameters

   i) An XRPD pattern comprising a peak at approximately 11 ° and 18°, and
   ii) A DSC scan showing a phase transition at 200°C - 220°C,
   **characterized in that** crystal form I is heated to below its melting point and then quenched to room temperature.

2. A process according to claim 1, wherein crystal form I is heated to below its melting point and then quenched to room temperature, **characterized in that** the crystal form I is heated up to between 205 °C and 210 °C.

3. A process for the transformation of crystal form II of astaxanthin into crystal form I of astaxanthin, wherein

   • crystal form I is **characterized by** the following parameters

   i) An XRPD (X-Ray Powder Diffraction) pattern comprising a peak between 20°and 21 °, and

ii) A DSC (Differential Scanning Calorimeter) scan showing a phase transition at 225 °C - 235°C; and wherein

• crystal form II is **characterized by** the following parameters

i) An XRPD pattern comprising a peak at approximately 11 ° and 18°, and
ii) A DSC scan showing a phase transition at 200°C - 220°C,
**characterized in that** the transformation is carried out by slurry conversion in dichloromethane or ethyl acetate.

**Patentansprüche**

1. Verfahren zur Transformation der Kristallform I von Astaxanthin in Kristallform II von Astaxanthin, wobei

• Kristallform I durch die folgenden Parameter gekennzeichnet ist:

i) ein X R P D-Muster (Röntgenpulverbeugungsmuster) mit einem Peak zwischen 20° und 21° und
ii) einen DSC-Scan (Differentialkalorimeter-Scan), der einen Phasenübergang bei 225°C - 235°C zeigt; und wobei

• Kristallform II durch die folgenden Parameter gekennzeichnet ist:

i) ein XRPD-Muster mit einem Peak zwischen ungefähr 11° und 18° und
ii) einen DSC-Scan, der einen Phasenübergang bei 200°C - 220°C zeigt;
**dadurch gekennzeichnet, dass** man Kristallform I auf eine Temperatur unterhalb ihres Schmelzpunkts erhitzt und dann auf Raumtemperatur abkühlt.

2. Verfahren nach Anspruch 1, wobei man Kristallform I auf eine Temperatur unterhalb ihres Schmelzpunkts erhitzt und dann auf Raumtemperatur abkühlt, **dadurch gekennzeichnet, dass** man die Kristallform I auf eine Temperatur zwischen 205°C und 210°C erhitzt.

3. Verfahren zur Transformation der Kristallform II von Astaxanthin in Kristallform I von Astaxanthin, wobei

• Kristallform I durch die folgenden Parameter gekennzeichnet ist:

i) ein X R P D-Muster (Röntgenpulverbeugungsmuster) mit einem Peak zwischen 20° und 21° und
ii) einen DSC-Scan (Differentialkalorimeter-Scan), der einen Phasenübergang bei 225°C - 235°C zeigt; und wobei

• Kristallform II durch die folgenden Parameter gekennzeichnet ist:

i) ein XRPD-Muster mit einem Peak zwischen ungefähr 11° und 18° und
ii) einen DSC-Scan, der einen Phasenübergang bei 200°C - 220°C zeigt;
**dadurch gekennzeichnet, dass** man die Transformation durch Aufschlämmungsumwandlung in Dichlormethan oder Essigsäureethylester durchführt.

**Revendications**

1. Procédé de transformation de la forme cristalline I d'astaxanthine en forme cristalline II d'astaxanthine, dans lequel

- la forme cristalline I est **caractérisée par** les paramètres suivants

i) un diagramme de XRPD (Diffraction sur Poudre de Rayons X) comprenant un pic entre 20° et 21° ; et
ii) un balayage par DSC (Calorimétrie à Balayage Différentiel) présentant une transition de phase à 225°C-235°C ; et dans lequel

- la forme cristalline II est **caractérisée par** les paramètres suivants

i) un diagramme de XRPD comprenant un pic à environ 11° et 18° ; et

ii) un balayage par DSC présentant une transition de phase à 200°C-220°C ;

**caractérisé en ce que** la forme cristalline I est chauffée jusqu'à une température inférieure à son point de fusion puis trempée jusqu'à température ambiante.

2. Procédé selon la revendication 1, dans lequel la forme cristalline I est chauffée jusqu'à une température inférieure à son point de fusion puis trempée jusqu'à température ambiante, **caractérisé en ce que** la forme cristalline I est chauffée jusqu'à une température comprise entre 205°C et 210°C.

3. Procédé de transformation de la forme cristalline II d'astaxanthine en forme cristalline I d'astaxanthine, dans lequel

- la forme cristalline I est **caractérisée par** les paramètres suivants

i) un diagramme de XRPD (Diffraction sur Poudre de Rayons X) comprenant un pic entre 20° et 21° ; et

ii) un balayage par DSC (Calorimétrie à Balayage Différentiel) présentant une transition de phase à 225°C-235°C ; et dans lequel

- la forme cristalline II est **caractérisée par** les paramètres suivants

i) un diagramme de XRPD comprenant un pic à environ 11° et 18° ; et

ii) un balayage par DSC présentant une transition de phase à 200°C-220°C ;

**caractérisé en ce que** la transformation est effectuée par une conversion en suspension dans du dichlorométhane ou de l'acétate d'éthyle.

**Figure 1**

**Figure 2**

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6863914 B
- US 6406735 B
- US 5364563 A
- US 6093348 A
- WO 2007020057 A
- US 5654488 A
- WO 891997 A
- EP 329754 A
- WO 03102116 A
- US 2861891 A
- US 6296877 B
- EP 564989 A